# EUROPEAN PATENT APPLICATION

(11) **EP 2 540 819 A1**
(43) Date of publication of application: **02.01.2013**
(21) Application number: 11171595.9
(22) Date of filing: 27.06.2011
(51) Int. Cl.: C12N 5/00

(54) **Method for cultivating cells in platelet-lysate-containing medium**

(71) Applicant: Rheinisch-Westfälische Technische Hochschule Aachen (RWTH), 52062 Aachen (DE)
(72) Inventor: Wagner, Wolfgang, Prof. Dr. Dr., 52078 Aachen (DE); Walenda, Gudrun, 52064 Aachen (DE)
(74) Representative: Remus, Alvaro Johannes

(57) **Abstract**

The invention concerns a method for cultivating cells 1 using a liquid medium 3 comprising blood platelet lysate and a gelatinization-inhibiting substance, said method comprising cultivating the cells 1 on the surface of and/or within a three-dimensional, gel-like substrate 4 comprising blood platelet lysate but being substantially devoid of a gelatinization-inhibiting substance, wherein the liquid medium 3 is layered on the gel-like substrate 4. The invention further relates to a two-phase system and a gel for cultivating cells.

## Description

### Background of the invention

The invention relates to a method for cultivating cells using a liquid medium comprising blood platelet lysate and a gelatinization-inhibiting substance. The invention further relates to a two-phase system and a gel for cultivating cells.

### Prior art

Cells are usually cultured in media that are supplemented with fetal calf serum (FCS) or other blood sera in order to provide the cells with essential growth factors. Although not any cell type actually needs serum for growth, most of the sensitive cell types, e.g., stem cells, are by now still dependent on serum-containing media. However, in light of therapeutic applications there is a growing interest to avoid the use of FCS due to potential xenogeneic immune reactions (e.g. anti-FCS antibodies), bovine pathogens (viruses and prions) and a high lotto-lot variability that hampers reproducibility of results (Heiskanen et al., 2007; Sundin et al., 2007). Chemically defined synthetic culture media are now commercially available, but their precise composition is often undisclosed and they often include other xenogeneic components such as recombinant growth factors.

Human platelet lysate (HPL) represents another attractive alternative to FCS. HPL can be generated from common platelet units by a simple freeze-thaw procedure. It is even possible to use HPL from the same donor to further minimize the risk of immunological side effects or viral infections. These studies were performed with mesenchymal stromal cells (MSC) from bone marrow and recently it has been demonstrated that HPL can also be used for isolation of MSC from human adipose tissue (Blande et al., 2009).

A method for preparing blood platelet lysate and a HPL medium is known from EP 0 413 727 B1. According to this known method citrate is added to whole blood in order to avoid coagulation. Plasma derived from this blood is centrifuged so as to obtain a platelet-rich paste and Ca²⁺ is then added for lysing the platelets and coagulating the remaining components. The filtered clear lysate is mixed with a conventional nutrient composition so as to obtain a HPL-containing medium.

Furthermore, WO 2008/034803 A1 discloses a cell culture medium supplement comprising plasma-free platelet lysate. The supplement further comprises additional substances such as albumin and dextran. In order to prepare the supplement, platelets are lysed by freezing and thawing them and acid citrate dextrose (ACD) or citrate phosphate dextrose (CPD) is added as anti-coagulant.

However, many cells, e.g., mesenchymal stromal cells (MSC), grow in a plastic adherent monolayer until they are contact inhibited. Sooner or later, the proliferation rate of the cells decays until they ultimately reach a senescent state. This is accompanied by enlarged morphology, reduced expression of surface markers and decreased differentiation potential. This process does not occur in parallel across cells in culture due to different subpopulations, apoptosis, and last but not least, contact inhibition within colonies. Lower seeding density facilitates higher intervals for cell passaging and hence, proliferation can only occur at the rim of the larger colonies. Consequently, when using plastic adherent monolayer cultures, it is hardly possible to accomplish a reliable expansion and to minimize contact inhibition. Moreover, cell passaging is not possible without using a cell-damaging peptidase treatment either. With stem cell cultures, it is another disadvantage of plastic adherent monolayer cultures that the plastic surface may cause undesired differentiation of the cells.

### Summary of the invention

It is the object of the invention to provide a method for cultivating cells using and a cell culture substrate that allows reliable expansion of cells, wherein contact inhibition is minimized and cell passaging is possible without needing an enzymatic treatment.

The object is achieved by providing a method comprising cultivating cells on the surface of and/or within a three-dimensional, gel-like substrate comprising blood platelet lysate (BPL) but being substantially devoid of a gelatinization-inhibiting substance, wherein a liquid medium comprising blood platelet lysate and a gelatinization-inhibiting substance is layered on the gel-like substrate. Gelatinization of pure blood platelet lysate or a composition comprising blood platelet lysate, which is free of any gelatinization-inhibiting substance such as heparin, occurs spontaneously at approximately 37°C within a few hours. Surprisingly, addition of coagulation-inducing agents, such as thrombin and/or calcium gluconate, is not necessary for gelatinization. Moreover, it turned out that the resulting gel-like substrate is a perfect substrate for expanding cells, especially mesenchymal stromal cells (MSC). The use of a gel-like substrate comprising blood platelet lysate avoids the risk of xenogenic immunoreactions or transmission of bovine pathogens and is also more efficient than FCS for large-scale expansion of cells, especially MSC from adipose tissue. For example, human platelet lysate (HPL) significantly increases the proliferation rate and maximal number of cumulative population doublings of adipose tissue derived MSC. Thus, the method according to the invention enables reliable expansion of cells and efficient minimization of contact inhibition as the cells can grow three-dimensionally within the substrate, i.e. multilayered, while growth factors are evenly available in all directions. Moreover, cell passaging is possible without the need of an enzymatic treatment, for example, a Trypsin/EDTA digestion, and a centrifugation step. As the gel-like substrate is biocompatible and hence provides a physiological environment for the cells, the risk of any undesired influence on the cells is minimized. For example, undesired differentiation of stem cells can be efficiently avoided. Further, covering the surface of the gel-like substrate with a liquid medium comprising blood platelet lysate and a gelatinization-inhibiting substance advantageously provides a homogenous environment for the cells and thus ensures optimal cultivation conditions.

In a preferred embodiment of the invention, the liquid medium and the gel-like substrate, beside the gelatinization-inhibiting substance, have a substantially identical composition, so that growth factors and nutrients are evenly distributed within the whole cultivation system and even the cells on the surface of the gel-like substrate can be cultivated under homogenous conditions.

In one embodiment of the invention, passaging or harvesting the cells is accomplished by enzymatic degradation of the gel-like substrate, preferably using a peptidase, in particular plasmin. Alternatively, passaging or harvesting the cells may be accomplished by mechanical fragmentation of the gel-like structure, preferably by pipetting, optionally followed by Trypsin/EDTA treatment or EDTA treatment for singularization of the cells. However, as even simple pipetting without any enzymatic treatment is sufficient with the method according to the invention, use of any enzymes during cell passaging can be avoided.

Preferably, the gelatinization-inhibiting substance comprises heparin.

In another preferred embodiment, both the liquid medium and the gel-like substrate are substantially devoid of serum so as to avoid any xenogeneic immune reactions or pathogenic infections. Thus, the method according to the invention is suitable for providing cells for therapeutic applications.

Preferably, the cells are stem or progenitor cells, particularly preferred mesenchymal stem cells, in particular mesenchymal stromal cells.

The object is further achieved by a method for producing a three-dimensional, gel-like substrate, said method comprising:
a) Providing a thrombocyte concentrate;
b) Harvesting blood platelets from said concentrate;
c) Lysing the blood platelets;
d) Adding at least one medium component to the lysate so as to obtain a medium comprising blood platelet lysate; and
e) Allowing the blood-platelet-lysate-medium to gelatinize at approximately 37°C so as to obtain a gel-like substrate comprising blood platelet lysate.

Surprisingly, it turned out that blood platelet lysate, either pure or diluted, spontaneously gelatinizes at approximately 37 °C without the necessity to add any coagulation-inducing agent such as, for example, thrombin and/or calcium gluconate.

In a preferred embodiment of this method, the medium component comprises a standardized medium composition for cell culture, preferably Dulbecco's Modified Eagles medium (DMEM). Thus, a medium containing essential growth factors and all necessary nutrients for cell cultures without undesired serum components is provided.

According to a preferred embodiment of the invention, the substrate may comprise 1-90 % blood platelet lysate, preferably 1-60 % blood platelet lysate, more preferred 5-40 % blood platelet lysate, in particular approximately 20 % blood platelet lysate. For example, the gel-like substrate according to the invention may essentially consist of 10 % HPL, 89 % DMEM, and 1 % Glutamin.

The object is further achieved by a two-phase system comprising (a) a liquid medium comprising blood platelet lysate and a gelatinization-inhibiting substance and (b) a three-dimensional, gel-like substrate comprising blood platelet lysate but being substantially devoid of a gelatinization-inhibiting substance.

In a preferred embodiment of this two-phase system, the liquid medium and the gel-like substrate, beside the gelatinization-inhibiting substance, have a substantially identical composition. Preferably, the gelatinization-inhibiting substance in the liquid phase of the two-phase system comprises heparin.

The object is also achieved by a gel-like substrate for cultivating cells, said gel-like substrate comprising blood platelet lysate and being substantially devoid of a gelatinization-inhibiting substance. Using this gel-like substrate, passaging of cells is possible without the need of an enzymatic treatment, for example, a Trypsin/EDTA digestion, and a centrifugation step. As the gel-like substrate according to the invention is biocompatible and hence provides a physiological environment for the cells, the risk of any undesired influence on the cells is minimized. Moreover, even simple pipetting without any enzymatic treatment is sufficient for passaging of cultured cells so that use of any enzymes during cell passaging can be avoided. Additionally, the gel-like substrate according to the invention is completely biodegradable so that it may be used in cell-therapeutic applications. Basically, the gel may essentially consist of undiluted blood platelet lysate. However, according to a preferred embodiment of the invention, the gel may comprise 1-90 % blood platelet lysate, preferably 1-60 % blood platelet lysate, more preferred 5-40 % blood platelet lysate, in particular approximately 20 % blood platelet lysate.

The gel-like substrate according to the invention can advantageously be used as a pharmaceutical composition in cell therapy. Particularly, cells, preferably stem cells, in particular mesenchymal stromal cells (MSC), embedded in the gel according to the invention may be transplanted into the body of a living animal or human for cell replacement, tissue repair, or the like. Alternatively, the gel-like substrate according to the invention may be transplanted and/or injected into an animal or human body in order to provide a suitable substrate for cells of the surrounding tissue or blood. The pharmaceutical composition according to the invention may be used for treating, e.g., arthrosis, necrosis, or other diseases correlated with damaged tissue.

One further aspect of the invention is the use of a gel comprising blood platelet lysate as a three-dimensional substrate for cultivating cells.

The invention is further described in detail with reference to the figures.

### Brief description of the figures

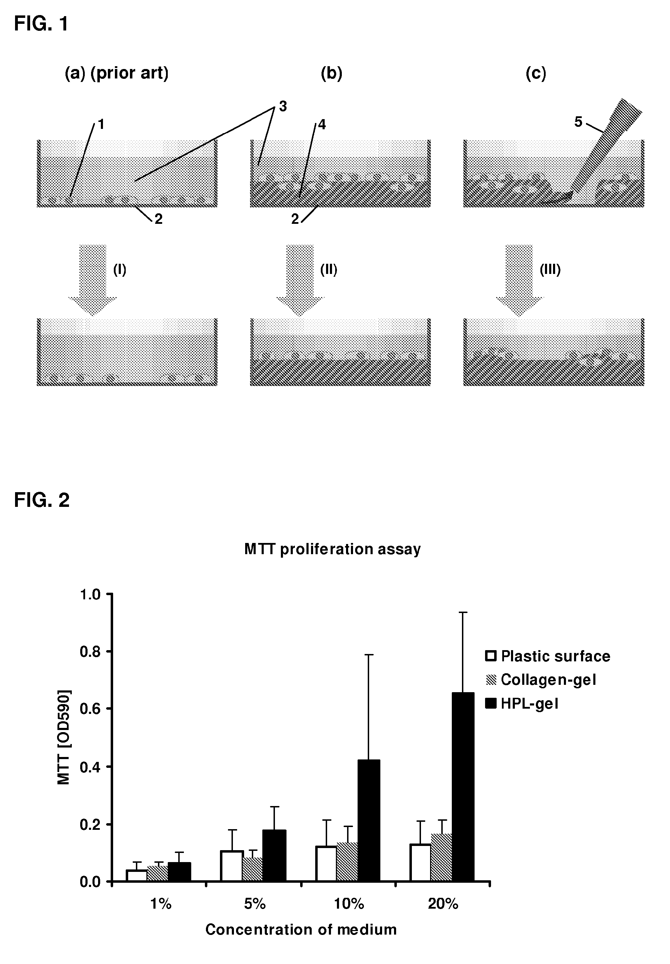

**Figure 1** shows a schematic representation of experimental arrangements for demonstrating the invention. Cells are cultivated either conventionally directly on cell culture plastics or on a gel according to the invention comprising human platelet lysate (HPL) but being devoid of heparin. The liquid culture medium contains essentially identical components but additionally includes heparin for inhibiting coagulation. The cells can be harvested by degradation of the HPL gel using plasmin or by pipetting.
**Figure 2** shows a bar diagram demonstrating the proliferation of mesenchymal stromal cells (MSCs) on different surfaces (white bars: plastic surface; grey bars: collagen gel; black bars: HPL gel). MSCs were cultured in culture medium comprising different concentrations of human platelet lysate (HPL) and the number of cells was determined after seven days using an MTT assay. The HPL concentration within the gel was respectively consistent with the HPL concentration in the liquid medium. Cell proliferation in HPL gel compared to proliferation on plastics and collagen gel shows significant differences (n=5).

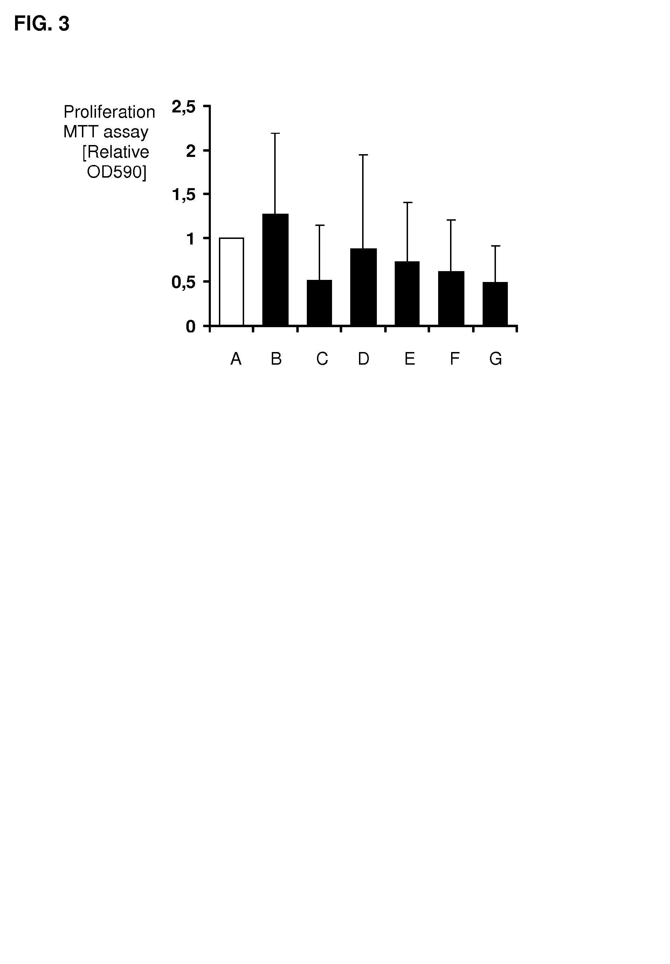

**Figure 3** shows a bar diagram demonstrating the proliferation of mesenchymal stromal cells (MSC) for different cell passaging methods with gel comprising human platelet lysate (HPL). MSC were cultivated for seven days on HPL gels and, for the sake of comparison, on cell culture plastics. Cells were harvested using plasmin treatment or pipetting, with or without subsequent Trypsin/EDTA or EDTA treatment, respectively, and then transferred to a new cell culture dish containing medium without HPL. After one further day of culturing, the number of cells was determined by a MTT assay. For the sake of control, the resulting values (black bars) were normed on cell culture plastic values (white bar, OD590 = 1). N=3, standard deviation is indicated.

- A:: Cultivation on plastics, passaging by Trypsin/EDTA
- B:: HPL gel, passaging by pipetting
- C:: HPL gel, passaging by pipetting and EDTA treatment
- D:: HPL gel, passaging by pipetting and Trypsin/EDTA treatment
- E:: HPL gel, passaging by plasmin degradation of the gel
- F:: HPL gel, passaging by plasmin degradation and EDTA treatment
- G:: HPL gel, passaging by plasmin degradation and Trypsin/EDTA treatment

### Description of exemplary and preferred embodiments of the invention

Mesenchymal stromal cells (MSC) are a biologically important cell population that is able to support hematopoiesis, can differentiate along mesenchymal and nonmesenchymal lineages *in vitro,* is capable of suppressing alloresponses, and appear to be nonimmunogenic. These properties suggest emerging roles for mesenchymal stromal cells in cell therapy.

MSC were isolated from the caput femoris upon hip fracture after written consent using guidelines approved by the Ethic Committee of the University of Aachen. Bone fragments were flushed with PBS and mononuclear cells (MNC) subsequently isolated by a density gradient on Biocoll (Biochrom AG, Berlin, Germany). Cells were cultured in fibronectin coated cell culture dishes (Greiner, Kremsmünster, Austria) in Dulbecco's Modified Eagles Medium-Low Glucose (DMEM-LG; PAA, Pasching, Austria) with 2 mM L-glutamine (Sigma Aldrich, St. Louis, MO, USA) and 100 U/mL penicilline/streptomycine (pen/strep; Lonza, Basel, Switzerland). Culture medium was supplemented with FCS (HyClone, Bonn, Germany) or HPL as indicated. Culture medium was always changed twice per week. When reaching 80% confluence cells were trypsinized, counted by in a Neubauer counting chamber (Brand, Wertheim, Germany) and replated at 10⁴ cells/cm². Cumulative population doublings were calculated from the first passage onward.

Alternatively, MSC may also be isolated from lipoaspirates.

Human platelet lysate (HPL) was generated from platelet units of healthy donors as described by Horn et al. (2010). In particular, HPL was generated by apheresis using the Trima Accel collection system (CaridianBCT, Garching, Germany). Such thrombocyte concentrates had a platelet content of 2.0 to 4.2×10¹¹ platelets in 200 mL plasma supplemented with Acid-Citrate-Dextrose (ACD) (1:11 v/v). Platelet units were aliquoted, twice frozen at -80°C, re-thawed at 37°C and centrifuged at 2600 g for 30 min at 4°C to remove cell fragments and pooled in equal amounts. The supernatant was filtered through 0.22 µm GD/X PVDF filter device (Whatman, Dassel, Germany), optionally supplemented with 2 U/mL heparin to avoid gelatinization, and stored at -80°C.

Cells were cultured in cell culture dishes (Greiner, Kremsmünster, Austria) in Dulbecco's Modified Eagles Medium-Low Glucose (DMEM-LG; PAA) with 2 mM L-glutamine (Sigma Aldrich, St. Louis, MO, USA) and 100 U/mL penicillin/streptomycin (pen/strep; Lonza, Basel, Switzerland). Culture medium/substrate was supplemented with FCS (HyClone, Bonn, Germany) or HPL as indicated in the specification. Cells were cultured at 37°C in a humidified atmosphere containing 5% carbon dioxide with medium changes twice per week.

Cell proliferation was evaluated via the Thiazolyl Blue Tetrazolium Bromide (MTT) assay. MSC were seeded on a 96-well plate (3,000 cells/well) in culture medium/substrate supplemented with 0, 1, 5, 10, or 20% of FCS or HPL as indicated in the specification. After 7 days of culture, cells were washed with PBS (PAA) and incubated with 1 mM MTT (Sigma-Aldrich) in PBS for 3.5 h. The excess solution was discarded and crystals were resolved in 4 mM HCl in isopropanol (both from Roth, Karlsruhe, Germany). Optical density (OD) was measured at wave length 590 nm with a Tecan Infinite 2000 plate reader (Tecan Trading, Männedorf, Switzerland).

**Figure 1** shows experimental arrangements for cultivating cells either directly on cell culture plastics (a) or on a gel according to the invention comprising human platelet lysate (HPL) but being devoid of heparin (b, c). In the prior art method according to (a), the cells 1 are directly attached to the tissue culture plastic 2 and cultured in a liquid HPL medium 3 containing heparin. According to this known method, the cells 1 are harvested by enzymatic treatment using Trypsin (I). In the method according to the invention (b, c), the cells are cultured on and within a gel-like substrate 4 which comprises HPL but is free of heparin. The liquid culture medium 3, which covers the substrate 4, contains essentially identical components but additionally includes heparin for inhibiting coagulation. The cells can be harvested either by degradation of the gel-like substrate 4 using plasmin (II) or by fragmentation of the gel-like substrate 4 using a pipet 5 and subsequent reseeding (III). As a result, cell passaging is possible without the need of an enzymatic treatment. As the gel-like substrate 4 is biocompatible and hence provides a physiological environment for the cells, the risk of any undesired influence on the cells by tissue culture plastic 2 is minimized. Further, covering the surface of the gel-like substrate 4 with a liquid medium 3 comprising blood platelet lysate and a gelatinization-inhibiting substance advantageously provides a homogenous environment for the cells and thus ensures optimal cultivation conditions.

**Figure 2** shows the proliferation of mesenchymal stromal cells (MSCs) on different surfaces. MSC were cultured in culture medium comprising different concentrations of human platelet lysate (HPL) and the number of cells was determined after seven days using an MTT assay. The HPL concentration within the gel was respectively consistent with the HPL concentration in the liquid medium. Cell proliferation in the HPL gel according to the invention (black bars) was significantly increased if compared to proliferation on plastics (white bars) or collagen gel (grey bars). The increase in proliferation is directly dependent on the concentration of the medium. Accordingly, human platelet lysate (HPL) significantly increases the proliferation rate of adipose tissue derived MSC. Thus, the method according to the invention enables reliable expansion of cells and efficient minimization of contact inhibition as the cells can grow three-dimensionally within the substrate, i.e. multilayered, while growth factors are evenly available in all directions.

**Figure 3** shows a bar diagram demonstrating the proliferation of mesenchymal stromal cells (MSC) for different cell passaging methods with gel comprising human platelet lysate (HPL). MSC were cultivated for seven days on HPL gels and, for the sake of comparison, on cell culture plastics. Cells were harvested using plasmin treatment or pipetting, with or without subsequent Trypsin/EDTA or EDTA treatment, respectively, and then transferred to a new cell culture dish containing medium without HPL. After one further day of culturing, the number of cells was determined by a MTT assay. The relative proliferation values show that cell passaging by simple pipetting without any enzymatic treatment is the most gentle way of detaching and reseeding the cells since cell damaging by enzymatic digestion is avoided.

### Non-patent literature

Blande, I. S.; Bassaneze, V.; Lavini-Ramos, C.; Fae, K. C.; Kalil, J.; Miyakawa, A. A.; Schettert, I. T.; Krieger, J. E.: Adipose tissue mesenchymal stem cell expansion in animal serum-free medium supplemented with autologous human platelet lysate. Transfusion 49: 2680-2685; 2009.

Heiskanen, A.; Satomaa, T.; Tiitinen, S.; Laitinen, A.; Mannelin, S.; Impola, U.; Mikkola, M.; Olsson, C.; Miller-Podraza, H.; Blomqvist, M.; Olonen, A.; Salo, H.; Lehenkari, P.; Tuuri, T.; Otonkoski, T.; Natunen, J.; Saarinen, J.; Laine, J.: N-glycolylneuraminic acid xenoantigen contamination of human embryonic and mesenchymal stem cells is substantially reversible. Stem Cells 25:197-202; 2007.

Horn, P.; Bokermann, G.; Cholewa, D.; Bork, S.; Walenda, T.; Koch, C.; Drescher, W.; Hutschenreuther, G.; Zenke, M.; Ho, A.; Wagner, W.: Comparison of Individual Platelet Lysates for Isolation of Human Mesenchymal Stromal Cells. Cytotherapy 12: 888-898; 2010.

Sundin, M.; Ringden, O.; Sundberg, B.; Nava, S.; Gotherstrom, C.; Le Blanc K.: No alloantibodies against mesenchymal stromal cells, but presence of anti-fetal calf serum antibodies, after transplantation in allogeneic hematopoietic stem cell recipients. Haematologica 92:1208-1215; 2007.

## Claims

1. Method for cultivating cells using a liquid medium comprising blood platelet lysate and a gelatinization-inhibiting substance, said method comprising cultivating the cells on the surface of and/or within a three-dimensional, gel-like substrate comprising blood platelet lysate but being substantially devoid of a gelatinization-inhibiting substance, wherein the liquid medium is layered on the gel-like substrate.

2. Method according to claim 1, wherein the liquid medium and the gel-like substrate, beside the gelatinization-inhibiting substance, have a substantially identical composition.

3. Method according to claim 1 or 2, wherein passaging or harvesting the cells is accomplished by enzymatic degradation of the gel-like substrate, preferably using a peptidase, in particular plasmin.

4. Method according to claim 1, 2 or 3, wherein passaging or harvesting the cells is accomplished by mechanical fragmentation of the gel-like structure, preferably by pipetting.

5. Method according to any one of claims 1 to 4, wherein the gelatinization-inhibiting substance comprises heparin.

6. Method according to any one of claims 1 to 5, wherein both the liquid medium and the gel-like substrate are substantially devoid of serum.

7. Method according to any one of claims 1 to 6, wherein said cells are stem or progenitor cells, preferably mesenchymal stem cells, in particular mesenchymal stromal cells.

8. A method for producing a three-dimensional, gel-like substrate, said method comprising:
- Providing a thrombocyte concentrate;
- Harvesting blood platelets from said concentrate;
- Lysing the blood platelets;
- Adding at least one medium component to the lysate so as to obtain a medium comprising blood platelet lysate; and
- Allowing the blood-platelet-lysate-medium to gelatinize at approximately 37 °C so as to obtain a gel-like substrate comprising blood platelet lysate.

9. Method according to claim 8, wherein said medium component comprises a standardized medium composition for cell culture, preferably DMEM medium.

10. Method according to claim 8 or 9, wherein said substrate comprises 1-90 % blood platelet lysate, preferably 1-60 % blood platelet lysate, more preferred 5-40 % blood platelet lysate, in particular approximately 20 % blood platelet lysate.

11. A two-phase system comprising (a) a liquid medium comprising blood platelet lysate and a gelatinization-inhibiting substance and (b) a three-dimensional, gel-like substrate comprising blood platelet lysate but being substantially devoid of a gelatinization-inhibiting substance.

12. The two-phase system according to claim 11, wherein the liquid medium and the gel-like substrate, beside the gelatinization-inhibiting substance, have a substantially identical composition.

13. A gel-like substrate for cultivating cells, said gel-like substrate comprising blood platelet lysate and being substantially devoid of a gelatinization-inhibiting substance.

14. A pharmaceutical composition comprising a gel-like substrate comprising blood platelet lysate and being substantially devoid of a gelatinization-inhibiting substance.

15. Use of a gel-like substrate comprising blood platelet lysate as a three-dimensional substrate for cultivating cells.
